(19) Europäisches Patentamt
European Patent Office
Office européen des brevets

(11) **EP 3 009 068 A1**

(12) **EUROPEAN PATENT APPLICATION**

(43) Date of publication:
**20.04.2016 Bulletin 2016/16**

(51) Int Cl.:
*A61B 5/00* (2006.01)  *A61B 5/0488* (2006.01)
*A61B 5/22* (2006.01)  *A63B 24/00* (2006.01)
*A61B 5/024* (2006.01)

(21) Application number: **15189870.7**

(22) Date of filing: **15.10.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **17.10.2014  KR 20140140975**

(71) Applicant: **Samsung Electronics Co., Ltd.
Gyeonggi-do, 443-742 (KR)**

(72) Inventors:
• **CHOI, Changmok
443-803 Gyeonggi-do (KR)**
• **KO, Byunghoon
443-803 Gyeonggi-do (KR)**
• **YOON, Seungkeun
443-803 Gyeonggi-do (KR)**

(74) Representative: **Grünecker Patent- und
Rechtsanwälte
PartG mbB
Leopoldstraße 4
80802 München (DE)**

(54) **METHOD AND APPARATUS FOR EXTRACTING ANAEROBIC THRESHOLD**

(57)    A method and apparatus for extracting an anaerobic threshold are provided. The apparatus may acquire an electromyogram (EMG) signal, and may extract an anaerobic threshold based on a change in the EMG signal. The apparatus may extract a heart rate corresponding to the anaerobic threshold as an anaerobic threshold heart rate.

FIG. 1

**Description**

**Field**

The following description relates to a method and apparatus for extracting an anaerobic threshold.

**Description of Related Art**

With improvement in standards of living, there has been an increase in interest in health care for improving a quality of life, and accordingly, medical services to manage health conditions are rapidly increasing. Due to rapid development of IT technologies along with the increase in demand for medical services, an interest in health care combined with medical technologies and IT technologies is rising. Accordingly, a large number of IT devices for providing a health care function and for providing health information are being developed. For example, a device to count steps of a user, or an amount of calories the user expends.

Research on technologies for providing health information suitable for a user by analyzing a health condition of the user has been actively conducted.

**SUMMARY**

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In one general aspect, there is provided an anaerobic threshold extraction apparatus including an electromyogram (EMG) signal acquirer configured to acquire an EMG signal, and an anaerobic threshold extractor configured to extract an anaerobic threshold based on a change in the EMG signal, the anaerobic threshold indicating a time when muscle fatigue occurs.

The EMG signal acquirer may be configured to acquire the EMG signal from at least one EMG sensor.

The anaerobic threshold extractor may be configured to extract the anaerobic threshold based on change in the EMG signal.

The EMG signal may change in response to an exercise load of a user gradually increasing to a threshold exercise load.

The the anaerobic threshold extractor may be further configured to transmit a control signal to an exercise equipment used by the user to gradually increase the exercise load to the threshold exercise load.

The anaerobic threshold extractor may be further configured to inform a user to gradually increase an exercise load to the threshold exercise load using any one or any combination of a visual interface, an acoustic interface, and a haptic interface.

The anaerobic threshold extractor may be further configured to extract a frequency of the EMG signal at predetermined time intervals, and to extract the anaerobic threshold based on a change in the frequency.

The anaerobic threshold extractor may be further configured to identify heart rates of the user from a heartbeat sensor, and to extract a heart rate corresponding to the anaerobic threshold as an anaerobic threshold heart rate.

The frequency comprises may be a median frequency or a mean frequency of the EMG signal.

The anaerobic threshold extractor may be further configured to identify when the muscle fatigue occurs, in response to the frequency being reduced by at least a set amount.

The anaerobic threshold extractor may be further configured to transform the EMG signal using a fast Fourier transform (FFT) or a short-time Fourier transform (STFT), and to extract the frequency from the transformed EMG signal.

The anaerobic threshold extractor may be further configured to extract the anaerobic threshold, based on a change in the EMG signal during a period of time or a change in the EMG signal until the exercise load reaches the threshold exercise load.

The anaerobic threshold extractor may be further configured to extract the anaerobic threshold based on an EMG signal corresponding to a largest reduction in a frequency of a plurality of EMG signals acquired from a plurality of EMG sensors.

The anaerobic threshold extractor may be further configured to transmit at least one of the extracted anaerobic threshold and the anaerobic threshold heart rate to an external apparatus via a communication interface.

In another general aspect, there is provided an exercise information providing apparatus, including an anaerobic threshold heart rate acquirer configured to acquire an anaerobic threshold heart rate based on a change in an electromyogram (EMG) signal, and an information providing unit configured to provide information on an exercise load corresponding to the anaerobic threshold heart rate.

The apparatus may including a heart rate identifier that may be configured to receive a heartbeat signal from a heartbeat sensor, and to identify heart rates based on the heartbeat signal.

The anaerobic threshold heart rate acquirer may be further configured to receive an anaerobic threshold from an apparatus via a communication interface, and to extract a heart rate corresponding to the anaerobic threshold as the anaerobic threshold heart rate.

The information providing unit may be further configured to inform a user on the exercise load using any one or any combination of a visual interface, an acoustic interface, and a haptic interface.

The information providing unit may be further configured to provide information on an exercise load to bring a heart rate within a threshold range of the anaerobic threshold heart rate, in response to the heart rate being outside the threshold range.

The exercise information providing apparatus may include a storage configured to store an anaerobic threshold heart rate of the user.

The information providing unit may be further configured to evaluate an exercise efficiency based on a ratio of a heart rate to the anaerobic threshold heart rate, and to provide the exercise efficiency.

In another general aspect, there is provided an anaerobic threshold heart rate extraction apparatus, including an electromyogram (EMG) sensor configured to sense an action potential of a muscle and to acquire an EMG signal, a heartbeat sensor configured to sense a heart rate and to acquire a heartbeat signal, an anaerobic threshold extractor configured to determine an anaerobic threshold based on a change in the EMG signal, the anaerobic threshold indicating a time when muscle fatigue occurs, and an anaerobic threshold heart rate extractor configured to extract a heart rate corresponding to the anaerobic threshold as an anaerobic threshold heart rate.

In another general aspect, there is provided a method for extracting anaerobic threshold, including acquiring an electromyogram (EMG) signal from at least one EMG sensor, determining the median or mean frequency of the acquired EMG signal, and extracting an anaerobic threshold based on the frequency corresponding to a change in the EMG signal.

The method may include identifying heart rates from a heartbeat sensor, and extracting a heart rate corresponding to the anaerobic threshold as an anaerobic threshold heart rate.

The determining of the median or mean frequency may include transforming the EMG signal using a fast Fourier transform (FFT) or a short-time Fourier transform (STFT) and extracting the frequency from the transformed EMG signal.

In another general aspect, there is provided an anaerobic threshold heart rate extraction apparatus including an EMG sensor configured to sense an action potential of a muscle and to acquire an EMG signal, a heartbeat sensor configured to sense a heart rate and to acquire a heartbeat signal, an anaerobic threshold extractor configured to extract a point in time at which muscle fatigue occurs as an anaerobic threshold, based on a change in the EMG signal, and an anaerobic threshold heart rate extractor configured to extract a heart rate corresponding to the anaerobic threshold as an anaerobic threshold heart rate, based on the heartbeat signal.

In another general aspect, there is provided an anaerobic threshold extraction method including acquiring an EMG signal of a user, and extracting an anaerobic threshold of the user, based on a change in the EMG signal.

In another general aspect, there is provided an exercise information providing method including acquiring an anaerobic threshold heart rate of a user extracted based on a change in an EMG signal of the user, and providing the user with information on an exercise load corresponding to the anaerobic threshold heart rate.

In a general aspect, there is provided an exercise information providing method including acquiring an EMG signal of a user, extracting an anaerobic threshold of the user, based on a change in the EMG signal, extracting a heart rate of the user corresponding to the anaerobic threshold as an anaerobic threshold heart rate, and providing the user with information on an exercise load corresponding to the anaerobic threshold heart rate.

Other features and aspects will be apparent from the following detailed description, the drawings, and the claims.

## BRIEF DESCRIPTION OF THE DRAWINGS

FIG. 1 is a diagram illustrating an example of an anaerobic threshold extraction apparatus.
FIG. 2 is a diagram illustrating an example of an exercise information providing apparatus.
FIG. 3 is a diagram illustrating an example of an anaerobic threshold extraction method.
FIG. 4 is a diagram illustrating an example of extraction of a frequency from an electromyogram (EMG) signal.
FIGS. 5 and 6 are diagrams illustrating an example of a comparison of an EMG signal corresponding to a contracted muscle of a user and an EMG signal corresponding to an uncontracted muscle.
FIGS. 7 and 8 illustrate an example of a method of extracting an anaerobic threshold from a plurality of EMG signals.
FIG. 9 illustrates an example of providing a user with exercise information using a plurality of apparatuses.
FIG. 10 illustrates another example of providing a user with exercise information using a plurality of apparatuses.
FIG. 11 illustrates examples of providing feedback through an exercise information providing apparatus.

FIG. 12 illustrates an example of an exercise history.

FIG. 13 is a block diagram illustrating another example of an exercise information providing apparatus.

FIG. 14 is a block diagram illustrating an example of an anaerobic threshold heart rate extraction apparatus.

FIG. 15 is a flowchart illustrating another example of an anaerobic threshold extraction method.

FIG. 16 is a flowchart illustrating an example of an exercise information providing method.

FIG. 17 is a flowchart illustrating another example of an exercise information providing method.

Throughout the drawings and the detailed description, unless otherwise described or provided, the same drawing reference numerals will be understood to refer to the same elements, features, and structures. The drawings may not be to scale, and the relative size, proportions, and depiction of elements in the drawings may be exaggerated for clarity, illustration, and convenience.

## DETAILED DESCRIPTION

The following detailed description is provided to assist the reader in gaining a comprehensive understanding of the methods, apparatuses, and/or systems described herein. However, various changes, modifications, and equivalents of the systems, apparatuses, and/or methods described herein will be apparent to one of ordinary skill in the art. The progression of processing steps and/or operations described is an example; however, the sequence of and/or operations is not limited to that set forth herein and may be changed as is known in the art, with the exception of steps and/or operations necessarily occurring in a certain order. Also, descriptions of functions and constructions that are well known to one of ordinary skill in the art may be omitted for increased clarity and conciseness.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "include" and/or "have," when used in this specification, specify the presence of stated features, integers, steps, operations, elements, components or combinations thereof, but do not preclude the presence or addition of one or more other features, integers, steps, operations, elements, components, and/or groups thereof.

As a non-exhaustive illustration only, an apparatus described herein may refer to mobile devices such as, for example, a cellular phone, a smart phone, a wearable smart device (such as, for example, a ring, a watch, a pair of glasses, glasses-type device, a bracelet, an ankle bracket, a belt, a necklace, an earring, a headband, a helmet, a device embedded in the cloths or the like), a personal computer (PC), a tablet personal computer (tablet), a phablet, a mobile internet device (MID), a personal digital assistant (PDA), an enterprise digital assistant (EDA), a head mount display (HMD) apparatus, a digital camera, a digital video camera, a portable game console, an MP3 player, a portable/personal multimedia player (PMP), a handheld e-book, an ultra mobile personal computer (UMPC), a portable lab-top PC, a global positioning system (GPS) navigation, a personal navigation device or portable navigation device (PND), a handheld game console, an e-book, and devices such as a high definition television (HDTV), an optical disc player, a DVD player, a Blue-ray player, a setup box, robot cleaners, or any other device capable of wireless communication or network communication consistent with that disclosed herein.

The wearable device may be self-mountable on the body of the user, such as, for example, the glasses or the bracelet. In another non-exhaustive example, the wearable device may be mounted on the body of the user through an attaching device, such as, for example, attaching a smart phone or a tablet to the arm of a user using an armband, or hanging the wearable device around the neck of a user using a lanyard.

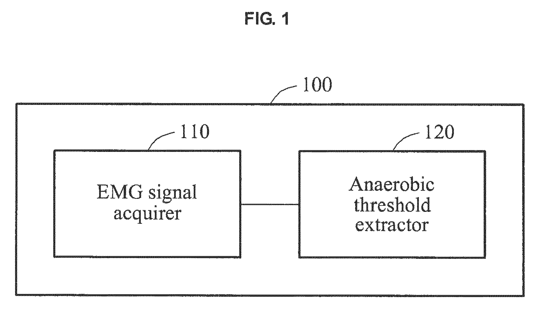

FIG. 1 illustrates an example of an anaerobic threshold extraction apparatus 100. Referring to FIG. 1, the anaerobic threshold extraction apparatus 100 includes an electromyogram (EMG) signal acquirer 110 and an anaerobic threshold extractor 120.

The EMG signal acquirer 110 acquires an EMG signal of a user. The EMG signal acquirer 110 may receive an EMG signal of a user in real time from at least one EMG sensor that senses an action potential of a muscle of the user. When a plurality of EMG sensors exist, the EMG signal acquirer 110 may acquire an EMG signal from each of the EMG sensors. The EMG signal acquirer 110 may include an analog-to-digital converter (ADC). The ADC may convert an EMG signal received from an EMG sensor to a digital signal.

The anaerobic threshold extractor 120 extracts an anaerobic threshold of the user, based on a change in the EMG signal. The anaerobic threshold may refer to a point when an aerobic exercise is switched to an anaerobic exercise. In a human body, fats may be mainly consumed during the aerobic exercise, and carbohydrates may be mainly consumed during the anaerobic exercise. Accordingly, when a user exercises while maintaining a heart rate at an anaerobic threshold when the aerobic exercise is switched to the anaerobic exercise, a largest amount of fats may be consumed.

The anaerobic threshold extractor 120 may extract an anaerobic threshold, based on a change in an EMG signal when an exercise load of the user increases to a predetermined threshold exercise load. Because the anaerobic threshold indicates a point when an aerobic exercise is switched to an anaerobic exercise, the anaerobic threshold extractor 120 may extract the anaerobic threshold while the exercise load is gradually increased from a lower exercise load to a higher exercise load.

In an example, the anaerobic threshold extractor 120 may control exercise equipment used by the user, to extract the anaerobic threshold. For example, the anaerobic threshold extractor 120 may transmit a control signal to the exercise equipment, using a communication interface. The control signal may gradually increase the exercise load of the user to a threshold exercise load. The communication interface may be an interface to communicate with an external apparatus. In description of FIGS. 1 through 17, the communication interface may include interfaces, such as, for example, a wireless Internet interface, and a near field communication (NFC) interface. The wireless Internet interface may include, for example, a wireless local area network (WLAN), a wireless fidelity (Wi-Fi), a Digital Living Network Alliance (DLNA), a Wireless Broadband (WiBro), a World Interoperability for Microwave Access (WiMAX), a High Speed Downlink Packet Access (HSDPA), and other interfaces known to one of ordinary skill in the art. The NFC interface may include, for example, a Bluetooth™, a radio frequency identification (RFID), an Infrared Data Association (IrDA), a Ultra Wideband (UWB), a ZigBee, an NFC, and other interfaces known to one of ordinary skill in the art. In addition, the communication interface may include, for example, all interfaces capable of capable of communication with external apparatuses, such as, for example, a wired interface.

For example, when the threshold exercise load is 12 kilometers per hour (km/h), the anaerobic threshold extractor 120 may transmit a control signal to gradually increase an exercise intensity of a treadmill used by a user from an exercise intensity of 1 km/h to an exercise intensity of 12 km/h during a period of time (for example, 20 minutes) to the treadmill via a communication interface.

In another example, to extract the anaerobic threshold, the anaerobic threshold extractor 120 may provide a user with information on an exercise load using at least one of a visual interface, such as, for example, a display, an acoustic interface, such as, for example, a speaker, or an audio output terminal and a haptic interface, such as, for example, a vibration motor. The user may gradually increase the exercise load may be to a threshold exercise load using the information. When a user wears the anaerobic threshold extraction apparatus 100, the anaerobic threshold extraction apparatus 100 may display, on a display of the anaerobic threshold extraction apparatus 100, an exercise intensity of an exercise needing to be performed by the user to extract an anaerobic threshold, may output the exercise intensity as voice commands through a speaker of the anaerobic threshold extraction apparatus 100, or may drive a vibration motor of the anaerobic threshold extraction apparatus 100 so that a vibration strength of the vibration motor may be increased based on the exercise load.

The anaerobic threshold extractor 120 may extract a frequency of the EMG signal at predetermined time intervals, and may extract a point in time when muscle fatigue of the user occurs, based on a change in the frequency. The anaerobic threshold extractor 120 may set the point in time when the muscle fatigue occurs as an anaerobic threshold. The frequency may refer to either a median frequency or a mean frequency of the EMG signal. The anaerobic threshold extractor 120 may perform a fast Fourier transform (FFT) or a short-time Fourier transform (STFT) on the EMG signal, and may extract a frequency from the EMG signal on which the FFT or STFT is performed. For example, the anaerobic threshold extractor 120 may perform the FFT on the EMG signal at predetermined time intervals (for example, 200 milliseconds (ms)) by applying a hamming window, and may extract a frequency from the EMG signal after FFT is performed. Additionally, the anaerobic threshold extractor 120 may extract a point in time when the frequency is reduced by an amount as the point in time when the muscle fatigue occurs. For example, the anaerobic threshold extractor 120 may extract a point in time when the frequency is reduced by at least 50 hertz (Hz) as the point in time when the muscle fatigue occurs. The anaerobic threshold extractor 120 may extract the point in time when the muscle fatigue occurs.

Muscle fibers forming human muscles are classified into a type I fiber, that is, a slow oxidative fiber, and a type II fiber, *i.e.,* a fast oxidative fiber and a fast glycolytic fiber. The type I fiber may contract when a small amount of force is generated, and the type II fiber may contract when a large amount of force is generated. When the type II fiber contracts, glucose may be broken down into adenosine triphosphate (ATP) energy and lactic acid. For example, when the type I fiber contracts, a muscle may not be fatigued. However, when the type II fiber contracts, a muscle may be fatigued due to a release of lactic acid. A reduction in a frequency of an EMG signal by may be caused by a release of lactic acid from the type II fiber. The anaerobic threshold extractor 120 may extract, as an anaerobic threshold the point at which the muscle fatigue occurs. When the muscle fatigue occurs, the type II fiber produces lactic acid causing the frequency of the EMG signal to be reduced. Thus, the anaerobic threshold extraction apparatus 100 may extract the anaerobic threshold, based on the EMG signal, instead of using blood of the user.

For example, when a plurality of EMG sensors exist, the anaerobic threshold extractor 120 may extract the anaerobic threshold, based on an EMG signal with a largest reduction in frequency of the EMG signals acquired from the EMG sensors.

Additionally, the anaerobic threshold extractor 120 may extract an anaerobic threshold heart rate, *i.e.,* a heart rate of the user corresponding to the anaerobic threshold. The anaerobic threshold extractor 120 may acquire a heartbeat signal of the user from a heartbeat sensor, such as, for example, a photoplethysmography (PPG) sensor. The anaerobic threshold extractor 120 may identify heart rates of the user at various time intervals. The anaerobic threshold extractor 120 may extract, as an anaerobic threshold heart rate, a heart rate corresponding to the anaerobic threshold from the identified heart rates. For example, when the anaerobic threshold corresponds to 11 minutes from when the user starts exercising, and a heart rate of the user at 11 minutes is "120," the anaerobic threshold extractor 120 may extract an anaerobic threshold heart rate of "120."

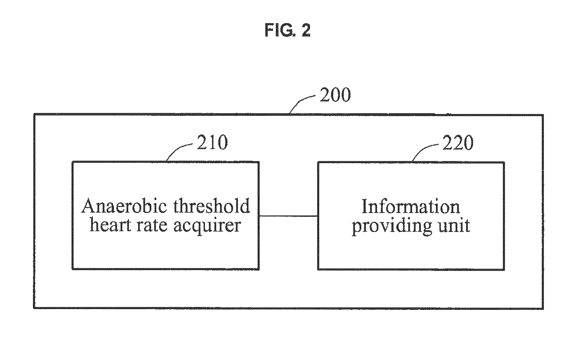

Furthermore, the anaerobic threshold extractor 120 may transmit at least one of the extracted anaerobic threshold and the extracted anaerobic threshold heart rate to an external apparatus via a communication interface. For example, the anaerobic threshold extractor 120 may transmit at least one of the extracted anaerobic threshold and the extracted anaerobic threshold heart rate to an exercise information providing apparatus 200 of FIG. 2 that will be described below. Additionally, the anaerobic threshold extractor 120 may transmit at least one of the extracted anaerobic threshold and the extracted anaerobic threshold heart rate to a wearable apparatus with an anaerobic threshold extraction apparatus, or a server configured to communicate with a wearable apparatus.

FIG. 2 illustrates an example of the exercise information providing apparatus 200. Referring to FIG. 2, the exercise information providing apparatus 200 includes an anaerobic threshold heart rate acquirer 210, and an information providing unit 220. the exercise information providing apparatus 200 may further include a heart rate identifier (not shown) to receive a heartbeat signal of a user from a heartbeat sensor, and to identify heart rates of the user based on the heartbeat signal.

The anaerobic threshold heart rate acquirer 210 acquires an anaerobic threshold heart rate of a user based on a change in an EMG signal of the user. The anaerobic threshold heart rate acquirer 210 may receive the anaerobic threshold heart rate from an external apparatus via a communication interface. In an example, the anaerobic threshold heart rate acquirer 210 may receive the anaerobic threshold heart rate from the anaerobic threshold extraction apparatus 100 of FIG. 1. In another example, the anaerobic threshold heart rate acquirer 210 may receive an anaerobic threshold of the user from an external apparatus via a communication interface, and may extract the anaerobic threshold heart rate, based on the anaerobic threshold. When the user extracts a heart rate threshold, the heart rate identifier may acquire a heartbeat signal of the user, may identify heart rates of the user based on an exercise time, and may extract, as an anaerobic threshold heart rate, a heart rate corresponding to an anaerobic threshold.

The information providing unit 220 provides the user with information on an exercise load corresponding to the anaerobic threshold heart rate. The information providing unit 220 may interfaces, such as, for example, a visual interface, an acoustic interface, and a haptic interface, to provide the user with the information on the exercise load. For example, when an exercise load corresponding to the anaerobic threshold heart rate is set to 9 km/h, the information providing unit 220 may display a message instructing the user to exercise at a speed of 9 km/h on a display of a wearable apparatus, or may output the message as voice commands through a speaker of the wearable apparatus. In this example, the information providing unit 220 may drive a vibration motor of the wearable apparatus to a rhythm corresponding to steps during an exercise at the speed of 9 km/h. Accordingly, an exercise may be performed at a pace corresponding to the anaerobic threshold heart rate and thus, the user may exercise at a maximum exercise efficiency.

When a heart rate of the user is beyond a threshold range of the anaerobic threshold heart rate, the information providing unit 220 may provide the user with information on an exercise load so that the heart beat is within the threshold range. For example, when an anaerobic threshold heart rate of a user is "110," a threshold range of the anaerobic threshold heart rate may be set as a range of "105" to "115." In this example, when a heart rate of the user is "100" during an exercise, the information providing unit 220 may provide the user with a message instructing the user to run faster. The information providing unit 220 may use interfaces, such as, for example, a visual interface, an acoustic interface, and a haptic interface to provide the information. In another example, when the heart rate of the user is "120" during the exercise, the information providing unit 220 may provide the user with a message instructing the user to run slower.

The information providing unit 220 may extract an exercise efficiency of the user by computing a ratio of a heart rate of the user to the anaerobic threshold heart rate, and may provide the user with the extracted exercise efficiency. For example, when the anaerobic threshold heart rate is set to "100" and when an average heart rate of "94" is measured for one hour during which the user exercises, an exercise efficiency of about 94% may be obtained, and the user may be provided with information indicating the exercise efficiency of about 94%.

The information providing unit 220 may include a storage unit (not shown) configured to store an anaerobic threshold heart rate of a user. The information providing unit 220 may provide the user with information on a change in the anaerobic threshold heart rate based on a time, using the anaerobic threshold heart rate stored in the storage unit. For example, an anaerobic threshold heart rate of "102" is acquired at January, an anaerobic threshold heart rate of "108" is acquired at February, an anaerobic threshold heart rate of "112" is acquired at March, and the anaerobic threshold heart rates are stored in the storage unit. The information providing unit 220 may extract the stored anaerobic threshold heart rates, and may provide the user with information on a change in the anaerobic threshold heart rates during a period of January to March.

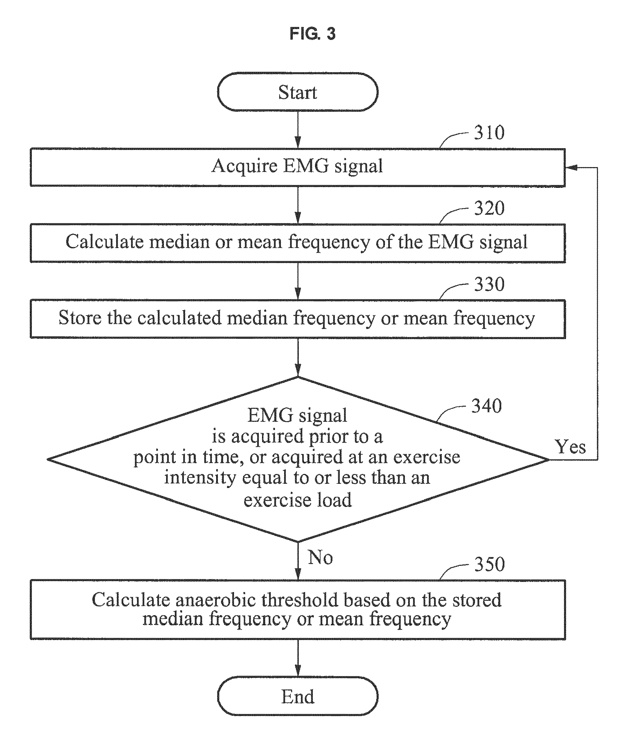

FIG. 3 illustrates an example of an anaerobic threshold extraction method. The operations in FIG. 3 may be performed in the sequence and manner as shown, although the order of some operations may be changed or some of the operations omitted without departing from the spirit and scope of the illustrative examples described. Many of the operations shown in FIG. 3 may be performed in parallel or concurrently. The above descriptions of FIGS. 1-2, is also applicable to FIG. 3, and is incorporated herein by reference. Thus, the above description may not be repeated here.

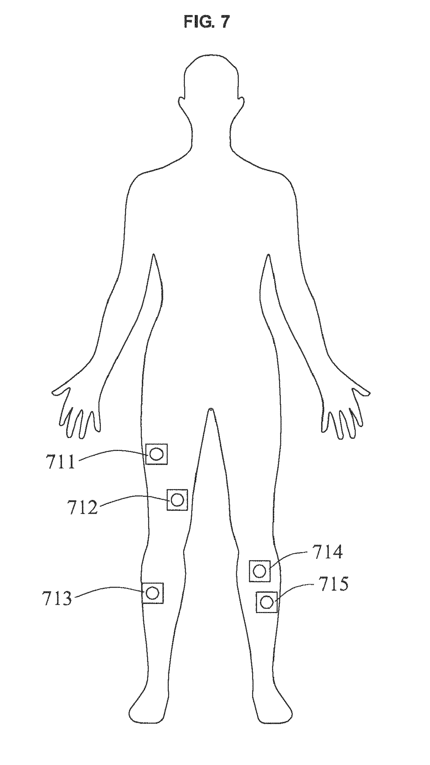

Referring to FIG. 3, in operation 310, an anaerobic threshold extraction apparatus acquires an EMG signal of a user. The anaerobic threshold extraction apparatus may receive an EMG signal of a user in real time from at least one EMG sensor. For example, the anaerobic threshold extraction apparatus may acquire an EMG signal from each of EMG sensors 711 through 715 of FIG. 7, which sense an action potential of a leg muscle of a user.

In operation 320, the anaerobic threshold extraction apparatus calculates a median frequency or a mean frequency of the EMG signal. The anaerobic threshold extraction apparatus may perform an FFT or an STFT on an EMG signal acquired in real time, and may convert the EMG signal from a time domain to a frequency domain. For example, when an EMG signal is received from an EMG sensor every 200 ms, the anaerobic threshold extraction apparatus may perform an FFT or an STFT on the EMG signal, and may calculate a median frequency or a mean frequency at an interval of 50 ms.

In operation 330, the anaerobic threshold extraction apparatus stores the calculated median frequency or the calculated mean frequency.

In operation 340, the anaerobic threshold extraction apparatus determines whether the EMG signal is acquired prior to a point in time, or acquired at an exercise intensity equal to or less than an exercise load. In an example, when the EMG signal is acquired prior to the point in time or acquired at the exercise intensity equal to or less than an exercise load, the anaerobic threshold extraction apparatus may repeatedly perform operations 310 through 340. In another example, when the EMG signal is not acquired prior to the point in time or acquired at the exercise intensity at an exercise intensity equal to or less than an exercise load, in operation 350, the anaerobic threshold extraction apparatus may calculate an anaerobic threshold, based on the stored median frequency or the stored mean frequency.

In operation 350, the anaerobic threshold extraction apparatus extracts a point in time when the median frequency or the mean frequency is reduced by at least a predetermined amount as a point in time when muscle fatigue occurs, and sets the point in time when the muscle fatigue occurs as an anaerobic threshold. For example, when the anaerobic threshold extraction apparatus is set to extract an anaerobic threshold based on an EMG signal during 15 minutes, the anaerobic threshold extraction apparatus may determine whether the EMG signal is acquired during 15 minutes from a point in time when the user starts exercising. In this example, when the EMG signal is determined to be acquired after 10 minutes from the point in time when the user starts exercising, the anaerobic threshold extraction apparatus may repeatedly perform operations 310 through 340. When the EMG signal is determined to be acquired after 15 minutes from the point in time when the user starts exercising, the anaerobic threshold extraction apparatus may calculate an anaerobic threshold based on a median frequency or a mean frequency of the EMG signal.

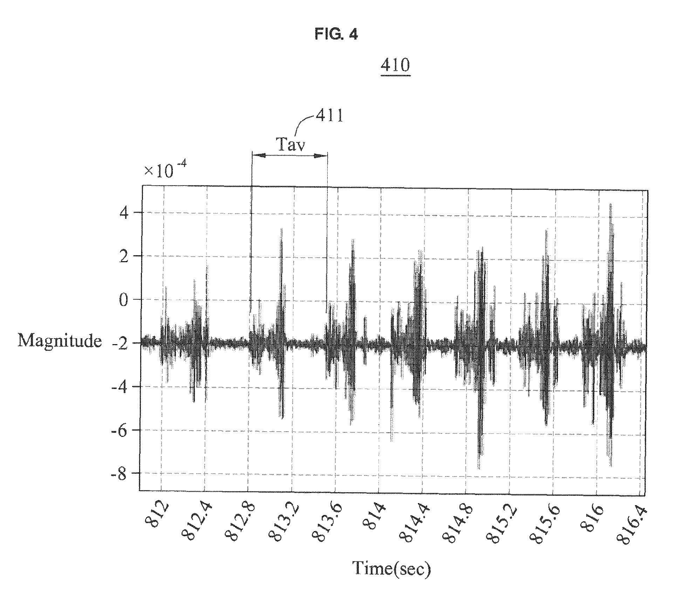

FIG. 4 illustrates an example of extraction of a frequency from an EMG signal. In FIG. 4, a graph 410 shows an EMG signal of a user. In the graph 410, a horizontal axis represents a time, and a vertical axis represents a magnitude (mV) of the EMG signal. In the graph 410, $T_{av}$ 411 represents a single period of the EMG signal.

An anaerobic threshold extraction apparatus may convert a time domain of the EMG signal to a frequency domain, and may extract a median frequency or a mean frequency from the EMG signal. For example, the anaerobic threshold extraction apparatus may perform an STFT on the EMG signal during the period $T_{av}$ 411, and may extract a frequency. In this example, the anaerobic threshold extraction apparatus may perform the STFT on the EMG signal, using Equation 1 shown below.

$$STFT_f^u(t', u) = \int_t [f(t) \cdot W(t - t')] \cdot e^{-j2\pi ut} dt$$

[Equation 1]

In Equation 1, t denotes a time parameter, u denotes a frequency parameter, f(t) denotes an EMG signal, and W(t-t') denotes a window, such as, for example, a hamming window. For example, the anaerobic threshold extraction apparatus may set a sampling rate to 1000 Hz, set a length of the window to 70 ms corresponding to 10% of the period $T_{av}$ 411, may set an overlap length of the window to 63 ms corresponding to 90% of the length of the window, and may perform the STFT on the EMG signal.

The anaerobic threshold extraction apparatus may calculate a median frequency or a mean frequency of the EMG signal converted to the frequency domain, and may extract the anaerobic threshold, based on the calculated median frequency or the calculated mean frequency.

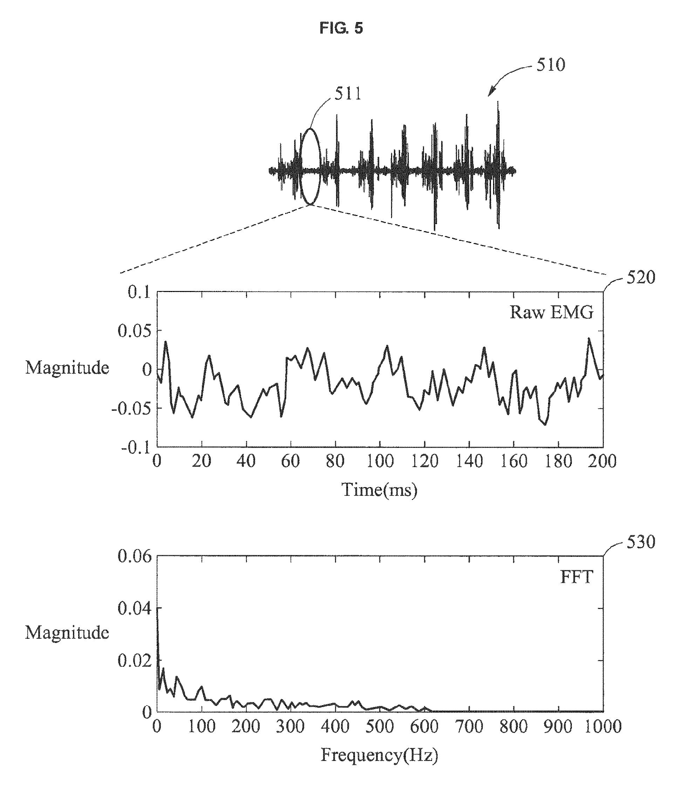

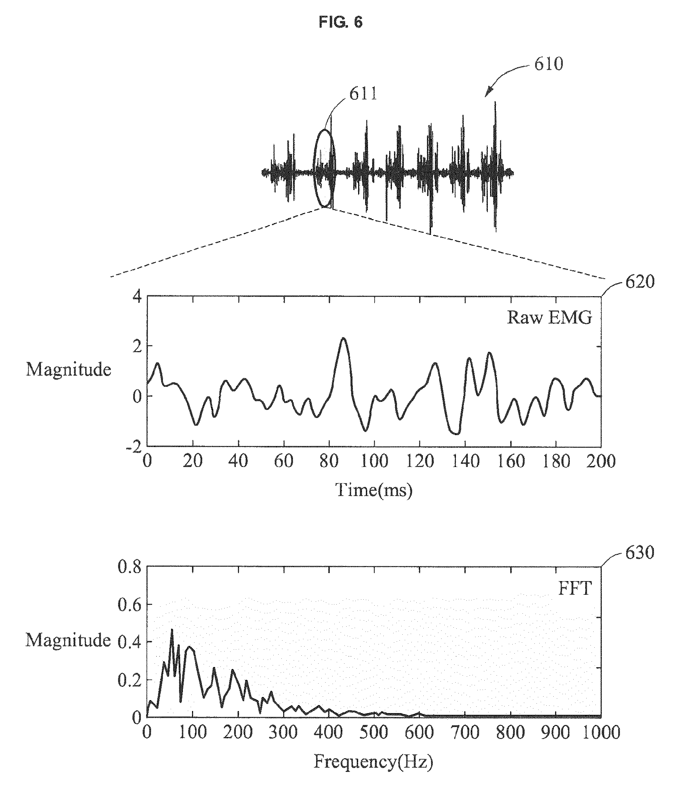

FIGS. 5 and 6 illustrate examples of a comparison of an EMG signal corresponding to a contracted muscle of a user and an EMG signal corresponding to an uncontracted muscle.

Referring to FIG. 5, in an entire EMG signal 510, an EMG signal 511 is measured when a muscle does not contract. Graph 520 shows the EMG signal 511 where a horizontal axis represents time, and a vertical axis represents magnitude (mV) of the EMG signal 511. Graph 530 showing the EMG signal 511 on which an FFT is performed, a horizontal axis represents frequency, and a vertical axis represents magnitude of the EMG signal 511.

Referring to FIG. 6, in an entire EMG signal 610, an EMG signal 611 is measured when a muscle contracts. Graph 620 shows the EMG signal 611 where a horizontal axis represents time, and a vertical axis represents magnitude (mV) of the EMG signal 611. Graph 630 showing the EMG signal 611 on which an FFT is performed, a horizontal axis represents frequency, and a vertical axis represents magnitude of the EMG signal 611.

In comparison between the graphs 520 and 620, in a time domain, the EMG signal 511 has a relatively small magnitude, that is, about ±0.05 mV, whereas the EMG signal 611 has a relatively large magnitude, that is, about ±2 mV. In comparison between the graphs 530 and 630, in a frequency domain, the EMG signal 511 has a relatively small magnitude, that is, about 0.02 mV, whereas the EMG signal 611 has a relatively large magnitude, that is, about 0. 4 mV. In addition, the EMG signal 511 has a frequency band of 0 Hz to 100 Hz, and the EMG signal 611 has a frequency band of 60 Hz to 200 Hz.

As described above, the EMG signals 510 and 610 differ from each other in magnitude and frequency band. Accordingly, the anaerobic threshold extraction apparatus may identify the EMG signal 610, and may extract an anaerobic threshold based on the EMG signal 610.

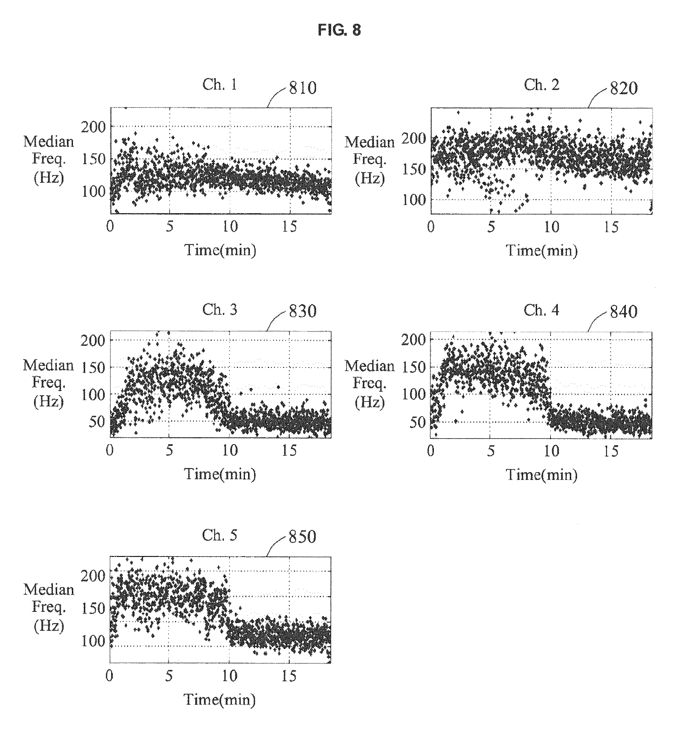

FIGS. 7 and 8 illustrate an example of a method of extracting an anaerobic threshold from a plurality of EMG signals. Referring to FIG. 7, to extract an anaerobic threshold, a plurality of EMG sensors, for example, the EMG sensors 711 through 715, may be attached to leg muscles of a user. The EMG sensors 711 through 715 may sense an action potential of each of muscles to which the EMG sensors 711 through 715 are attached, may generate an EMG signal, and may transmit the generated EMG signal to an anaerobic threshold extraction apparatus.

Referring to FIG. 8, graphs 810 through 850 show EMG signals that are measured for 20 minutes from when a user starts exercising and that are received from the EMG sensors 711 through 715, respectively. In each of the graphs 810 through 850, a horizontal axis represents time, and a vertical axis represents magnitude of a median frequency.

An anaerobic threshold extraction apparatus may extract an anaerobic threshold, based on an EMG signal acquired from one of the EMG sensors 711 through 715. For example, the anaerobic threshold extraction apparatus may extract an anaerobic threshold, based on an EMG signal corresponding to a largest amount of a frequency to be reduced among EMG signals acquired from the EMG sensors 711 through 715. The graph 840 shows a largest amount of reduction in a median frequency of an EMG signal acquired from the EMG sensor 714. Accordingly, the anaerobic threshold extraction apparatus may extract an anaerobic threshold of a user, based on the EMG signal acquired from the EMG sensor 714.

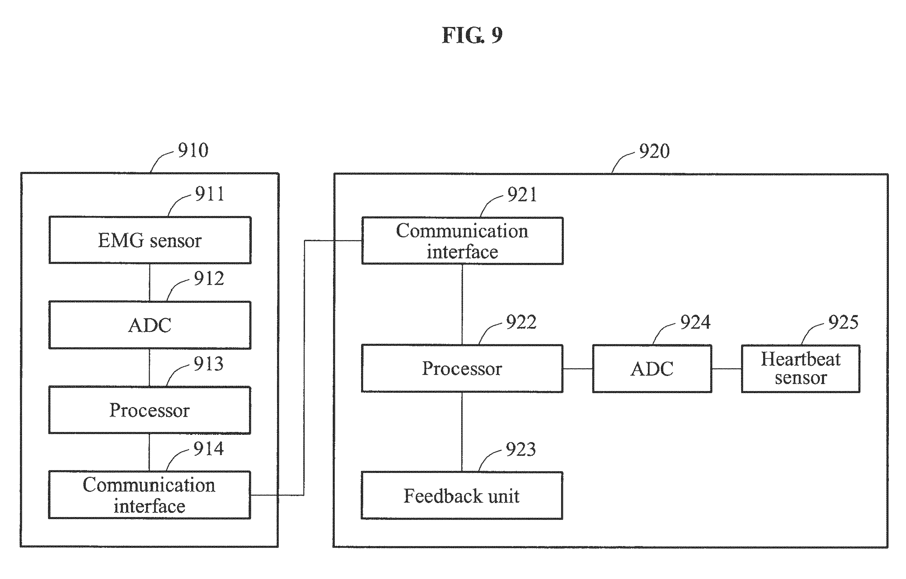

FIG. 9 illustrates an example of providing a user with exercise information using a plurality of apparatuses. Referring to FIG. 9, a first apparatus 910 includes an EMG sensor 911, an ADC 912, a processor 913, and a communication interface 914. A second apparatus 920 includes a communication interface 921, a processor 922, a feedback unit 923, an ADC 924, and a heartbeat sensor 925.

The EMG sensor 911 may be attached to a muscle of the user, to sense an action potential of the muscle, to generate an EMG signal, and to transmit the generated EMG signal to the ADC 912. The ADC 912 may convert the EMG signal received from the EMG sensor 911 to a digital signal. The processor 913 may extract an anaerobic threshold of the user, based on a change in the EMG signal.

The processor 913 may perform an FFT or an STFT on the EMG signal based on an exercise load of the user that is gradually increased to a threshold exercise load. The processor 913 may extract a frequency at predetermined time intervals, and may extract the anaerobic threshold based on a change in the frequency. The anaerobic threshold is a point in time when muscle fatigue of the user occurs. The frequency may be, for example, a median frequency or a standard frequency. The processor 913 may extract, as an anaerobic threshold, a point in time when a median frequency or a standard frequency is reduced by at least a predetermined amount.

The first apparatus 910 may extract an anaerobic threshold heart rate, *i.e.,* a heart rate of the user corresponding to the anaerobic threshold. For example, to estimate the anaerobic threshold heart rate, the heartbeat sensor 925 may sense a heartbeat of the user from a point in time when the user starts exercising, and may generate a heartbeat signal. The processor 922 may transmit the heartbeat signal to the first apparatus 910 via the communication interface 921. The processor 913 may identify heart rates of the user based on an exercise time, using the heartbeat signal received from the second apparatus 920. The processor 913 may extract, as the anaerobic threshold heart rate, a heart rate corresponding to the anaerobic threshold from the identified heart rates.

The processor 913 may transmit at least one of the EMG signal, the anaerobic threshold, and the anaerobic threshold heart rate to the second apparatus 920, using the communication interface 914.

The heartbeat sensor 925 may sense a heartbeat of the user, and may generate a heartbeat signal. The ADC 924 may convert the heartbeat signal from an analog to a digital form. The processor 922 may identify heart rates of the user, using the heartbeat signal.

Additional, the processor 922 may receive at least one of the EMG signal, the anaerobic threshold and the anaerobic threshold heart rate via the communication interface 921 from the first apparatus 910.

When the EMG signal is received from the first apparatus 910, the processor 922 may extract the anaerobic threshold, based on the EMG signal. For example, the processor 913 in the first apparatus 910 may transmit the EMG signal converted to the digital form by the ADC 912 to the second apparatus 920, using the communication interface 914. The processor 922 in the second apparatus 920 may extract the anaerobic threshold, based on a change in the EMG signal received from the first apparatus 910.

When the anaerobic threshold is extracted, or received from the first apparatus 910, the processor 922 may extract the anaerobic threshold heart rate. For example, the heartbeat sensor 925 may sense a heartbeat of the user from a point in time when the user starts exercising, and may generate a heartbeat signal, to estimate the anaerobic threshold heart rate. The processor 922 may identify heart rates of the user based on an exercise time, using the heartbeat signal, and may extract, as the anaerobic threshold heart rate, a heart rate corresponding to the anaerobic threshold among the identified heart rates.

The feedback unit 923 may include an interface, such as, for example, a visual interface, an acoustic interface, and a haptic interface to communicate with the user. For example, the visual interface may be a display of the second apparatus 920, the acoustic interface may be a speaker or an audio output terminal of the second apparatus 920, and the haptic interface may be a vibration motor of the second apparatus 920.

The processor 922 may provide the user with information on an exercise load corresponding to the anaerobic threshold heart rate, through the feedback unit 923. For example, the processor 922 may receive a heartbeat signal from the heartbeat sensor 925 when the user is exercising, and may identify heart rates of the user during an exercise time, based on the received heartbeat signal. When a heart rate is beyond a threshold range of the anaerobic threshold heart rate, the processor 922 may provide the user with information on an exercise load to bring the heart rate within the threshold range, through the feedback unit 923.

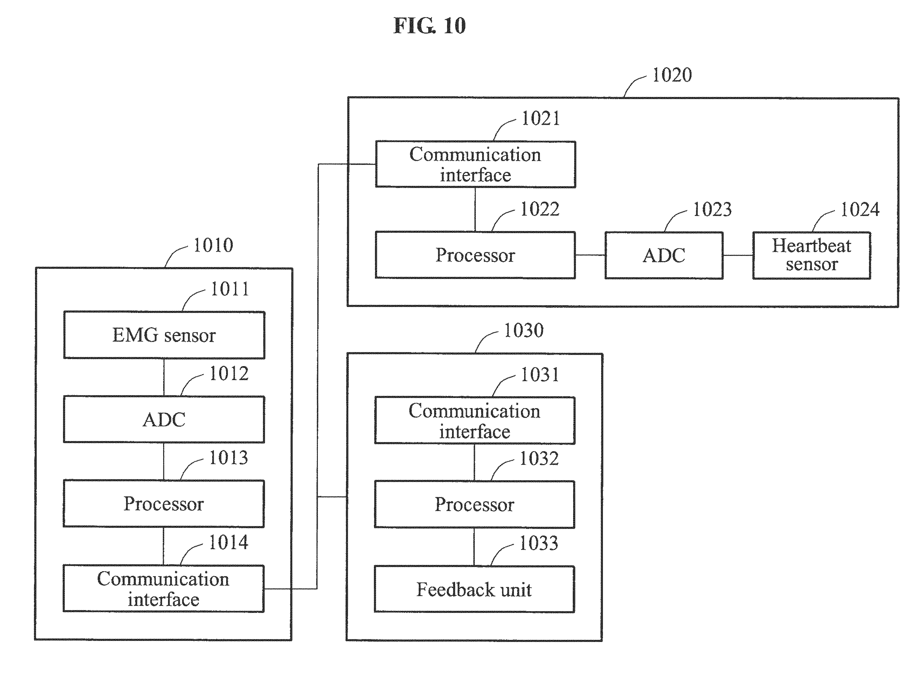

FIG. 10 illustrates another example of providing a user with exercise information using a plurality of apparatuses.

Referring to FIG. 10, a first apparatus 1010 includes an EMG sensor 1011, an ADC 1012, a processor 1013, and a communication interface 1014. A second apparatus 1020 includes a communication interface 1021, a processor 1022, an ADC 1023, and a heartbeat sensor 1024. A third apparatus 1030 includes a communication interface 1031, a processor 1032, and a feedback unit 1033.

The EMG sensor 1011 may be attached to a muscle of the user, to sense an action potential of the muscle, to generate an EMG signal, and to transmit the generated EMG signal to the ADC 1012. The ADC 1012 may convert the EMG signal received from the EMG sensor 1011 to a digital signal. The processor 1013 may transmit the EMG signal to the second apparatus 1020 or the third apparatus 1030, via the communication interface 1014.

The heartbeat sensor 1024 may sense a heartbeat of the user, and may generate a heartbeat signal. The ADC 1023 may convert the heartbeat signal from an analog to a digital form. The heartbeat of the user may be sensed in both an example in which a user exercises to extract an anaerobic threshold and an example in which a user exercises regardless of extraction of an anaerobic threshold. The processor 1022 may identify heart rates of the user, using the heartbeat signal. Additionally, the processor 1022 may transmit heart rates of the user or the heart signal to the first apparatus 1010 or the third apparatus 1030 via the communication interface 1021.

The processor 1032 may receive the EMG signal from the first apparatus 1010, and may receive the heart rates or the heart signal from the second apparatus 1020, through the communication interface 1031.

The processor 1032 may perform an FFT or an STFT on the EMG signal based on an exercise load of the user that is gradually increased to a threshold exercise load. The processor 1032 may extract a frequency at predetermined time intervals, and may extract the anaerobic threshold based on a change in the frequency. The anaerobic threshold is a point in time when muscle fatigue of the user occurs. The frequency may be, for example, a median frequency or a standard frequency. The processor 1032 may extract, as an anaerobic threshold, a point in time when a median frequency or a standard frequency is reduced by at least a predetermined amount.

Additionally, the processor 1032 may extract an anaerobic threshold heart rate, *i.e.,* a heart rate of the user corresponding to the anaerobic threshold. For example, the processor 1032 may receive the heart rates from the second apparatus 1020 via the communication interface 1031, may identify the heart rates based on the exercise time, and may extract, as the anaerobic threshold heart rate, a heart rate corresponding to the anaerobic threshold.

The feedback unit 1033 may include an interface, such as, for example, a visual interface, an acoustic interface, and a haptic interface. For example, the visual interface may be a display of the third apparatus 1030, the acoustic interface may be a speaker or an audio output terminal of the third apparatus 1030, and the haptic interface may be a vibration motor of the third apparatus 1030.

The processor 1032 may provide the user with information on an exercise load corresponding to the anaerobic threshold heart rate, through the feedback unit 1033. For example, while the user exercises, the processor 1032 may receive a heart rate of a user from the second apparatus 1020 via the communication interface 1031, and may identify heart rates of the user during an exercise time. When a heart rate of the user is beyond a threshold range of the anaerobic threshold heart rate, the processor 1032 may provide the user with information on an exercise load to bring the heart rate within the threshold range, through the feedback unit 1033.

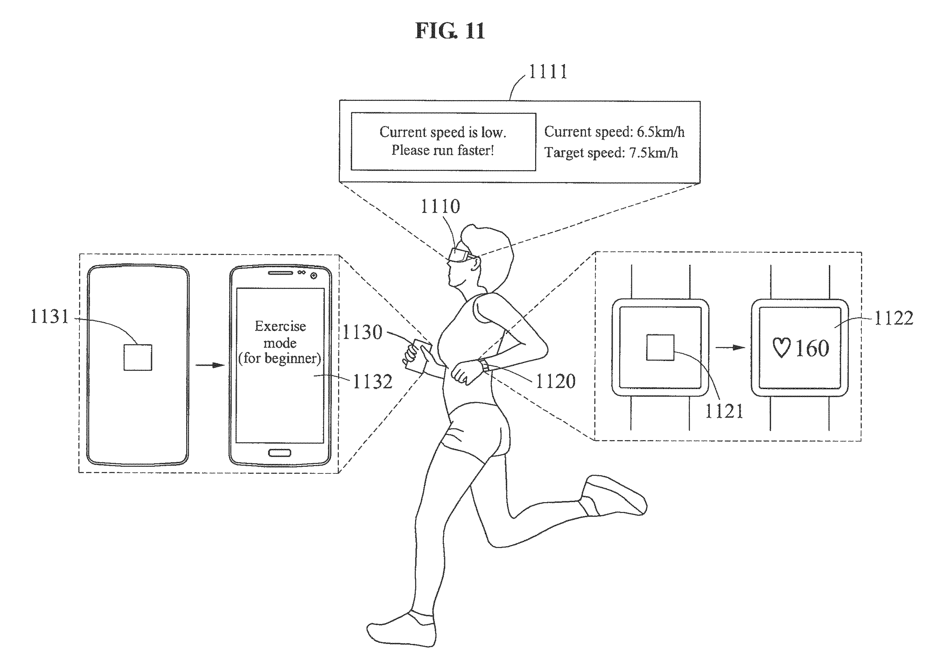

FIG. 11 illustrates examples of providing feedback through an exercise information providing apparatus. Referring to FIG. 11, a first exercise information providing apparatus 1110, a second exercise information providing apparatus 1120, and a third exercise information providing apparatus 1130 may provide a user with exercise information so that the user may exercise based on an anaerobic threshold heart rate. For example, the first exercise information providing apparatus 1110 may be an HMD apparatus, the second exercise information providing apparatus 1120 may be a wearable apparatus, and the third exercise information providing apparatus 1130 may be a mobile terminal.

The user may switch an operating mode of one or more of the first exercise information providing apparatus 1110 through the third exercise information providing apparatus 1130 to an exercise mode. For example, when the user switches an operating mode of the third exercise information providing apparatus 1130 to the exercise mode, the third exercise information providing apparatus 1130 may transmit a control signal to each of the first exercise information providing apparatus 1110 and the second exercise information providing apparatus 1120. The control signal may switch an operating mode of each of the first exercise information providing apparatus 1110 and the second exercise information providing apparatus 1120 to the exercise mode. At least one of the first exercise information providing apparatus 1110 through the third exercise information providing apparatus 1130 may store, in advance, an anaerobic threshold heart rate of the user.

In the exercise mode, the second exercise information providing apparatus 1120 or the third exercise information providing apparatus 1130 may identify heart rates of the user, using a heartbeat sensor 1121 or 1131. The heartbeat sensors 1121 and 1131 may be disposed on a rear side of the second exercise information providing apparatus 1120 and a rear side of the third exercise information providing apparatus 1130, respectively.

The first exercise information providing apparatus 1110 through the third exercise information providing apparatus 1130 may provide the user with information on an exercise load corresponding to the anaerobic threshold heart rate. For example, when an exercise load corresponding to an anaerobic threshold heart rate is 8 km/h, the first exercise information providing apparatus 1110 may display, on a display 1111, a current speed of the user and a speed corresponding to the anaerobic threshold heart rate. The first exercise information providing apparatus 1110 may inform the user of the current speed using a voice output terminal. In this example, the second exercise information providing apparatus 1120 may display a heart rate of the user on a display 1122, and the third exercise information providing apparatus 1130 may display, on a display 1132, information indicating that the third exercise information providing apparatus 1130 is being in the exercise mode.

When a heart rate of the user is beyond a threshold range of the anaerobic threshold heart rate, the first exercise information providing apparatus 1110 through the third exercise information providing apparatus 1130 may provide the user with information on an exercise load to bring the heart rate within the threshold range. For example, when an anaerobic threshold heart rate of the user is "120," a threshold range of the anaerobic threshold heart rate may be set as a range of "115" to "125." In this example, when a heart rate of the user is "100" during an exercise, the first exercise information providing apparatus 1110 may display a message instructing the user to run faster on the display 1111, and the second exercise information providing apparatus 1120 may drive a vibration motor of the second exercise information providing apparatus 1120 to a rhythm corresponding to the heart rate of "120." Additionally, the third exercise information providing apparatus 1130 may output a message instructing the user to run faster through a speaker.

In another example, when the user switches an operating mode of the third exercise information providing apparatus 1130 to the exercise mode, the third exercise information providing apparatus 1130 may transmit a control signal to the first exercise information providing apparatus 1110 to switch an operating mode of the first exercise information providing apparatus 1110 to the exercise mode.

In another example, when the user switches an operating mode of the third exercise information providing apparatus 1130 to the exercise mode, the third exercise information providing apparatus 1130 may not transmit a control signal to either the first exercise information providing apparatus 1110 or the second exercise information providing apparatus 1120. Thus, the first exercise information providing apparatus 1110 and the second exercise information providing apparatus 1120 may not be switched to an the exercise mode.

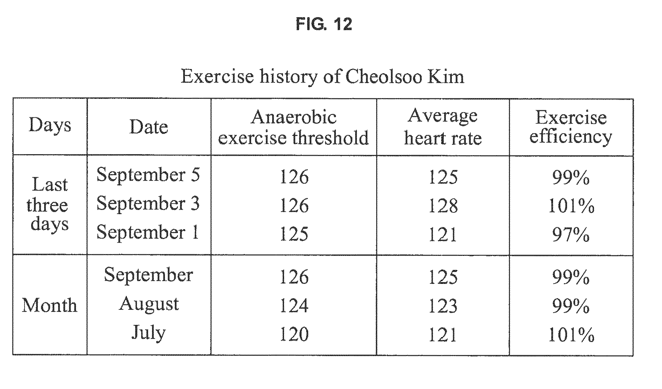

FIG. 12 illustrates an example of an exercise history. Referring to FIG. 12, an exercise information providing apparatus may store an anaerobic threshold heart rate of a user, and a heart rate of the user, which are measured when the user exercises. The exercise information providing apparatus may compute a ratio of the heart rate to the anaerobic threshold heart rate to derive an exercise efficiency of the user, and may provide the user with the extracted exercise efficiency. For example, when the anaerobic threshold heart rate is "126" and an average heart rate for one hour during which the user exercises is "125," an exercise efficiency may be about 99%. In the example of FIG. 12, the exercise information providing apparatus may daily or monthly provide the user with the user's exercise efficiency. Additionally, the exercise information providing apparatus may store the anaerobic threshold heart rate, and an exercise history recorded when the user exercises, and may construct a database for the user, based on the stored anaerobic threshold heart rate and the stored exercise history. The exercise history may include, for example, an exercise time, or an exercise efficiency and a heart rate of the user measured when the user exercises.

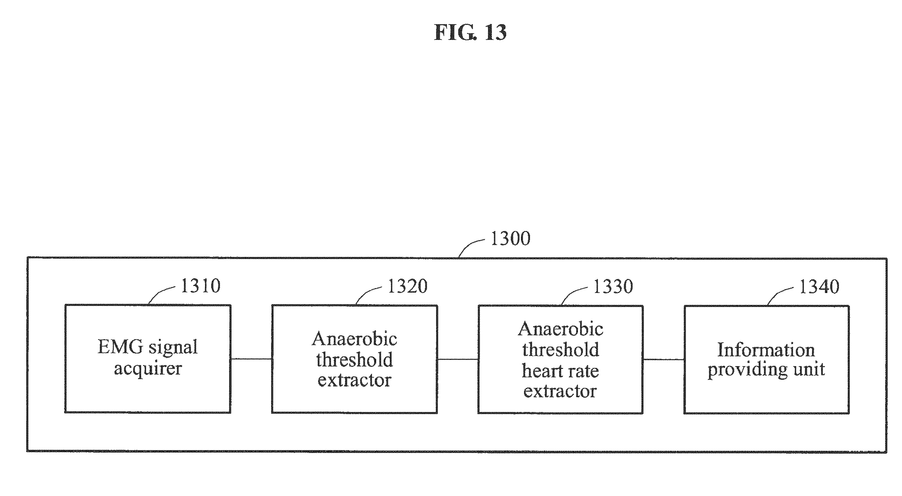

FIG. 13 illustrates another example of an exercise information providing apparatus 1300. Referring to FIG. 13, the exercise information providing apparatus 1300 includes an EMG signal acquirer 1310, an anaerobic threshold extractor 1320, an anaerobic threshold heart rate extractor 1330, and an information providing unit 1340.

The EMG signal acquirer 1310 acquires an EMG signal of a user. The anaerobic threshold extractor 1320 extracts an anaerobic threshold of the user, based on a change in the EMG signal. The anaerobic threshold heart rate extractor 1330 extracts a heart rate of the user corresponding to the anaerobic threshold as an anaerobic threshold heart rate. The information providing unit 1340 provides the user with information on an exercise load corresponding to the anaerobic threshold heart rate.

The above description of FIGS. 1 through 12 is also applicable to the exercise information providing apparatus 1300 of FIG. 13, and is incorporated herein by reference. Thus, the above description may not be repeated here.

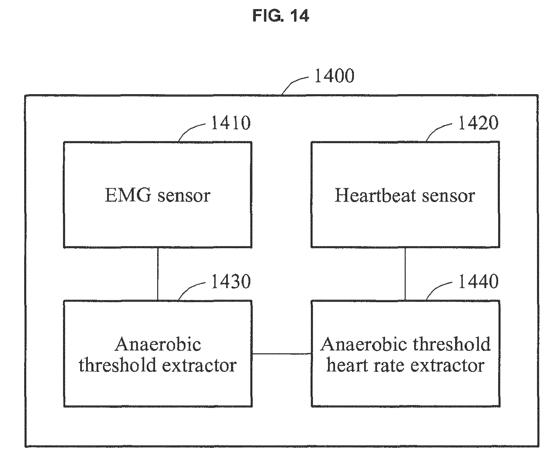

FIG. 14 illustrates an example of an anaerobic threshold heart rate extraction apparatus 1400. Referring to FIG. 14, the anaerobic threshold heart rate extraction apparatus 1400 includes an EMG sensor 1410, a heartbeat sensor 1420, an anaerobic threshold extractor 1430, and an anaerobic threshold heart rate extractor 1440.

The EMG sensor 1410 senses an action potential of a muscle of a user, and acquires an EMG signal.

The heartbeat sensor 1420 senses a heart rate of the user, and acquires a heartbeat signal. The anaerobic threshold extractor 1430 extracts, as an anaerobic threshold of the user, a point in time when muscle fatigue of the user occurs, based on a change in the EMG signal. The anaerobic threshold heart rate extractor 1440 extracts a heart rate corresponding to the anaerobic threshold as an anaerobic threshold heart rate.

The above description of FIGS. 1 through 13 is also applicable to the anaerobic threshold heart rate extraction apparatus 1400 of FIG. 14, and is incorporated herein by reference. Thus, the above description may not be repeated here.

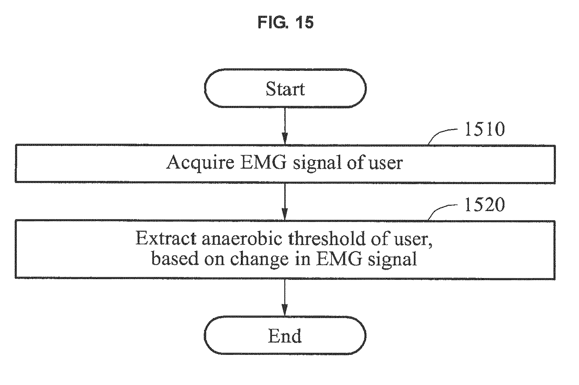

FIG. 15 illustrates another example of an anaerobic threshold extraction method. The operations in FIG. 15 may be performed in the sequence and manner as shown, although the order of some operations may be changed or some of the operations omitted without departing from the scope of the illustrative examples described. Many of the operations shown in FIG. 15 may be performed in parallel or concurrently. The above descriptions of FIGS. 1-14, is also applicable to FIG. 15. Thus, the above description may not be repeated here.

Referring to FIG. 15, in operation 1510, an anaerobic threshold extraction apparatus acquires an EMG signal of a user. In operation 1520, the anaerobic threshold extraction apparatus extracts an anaerobic threshold of the user, based on a change in the EMG signal.

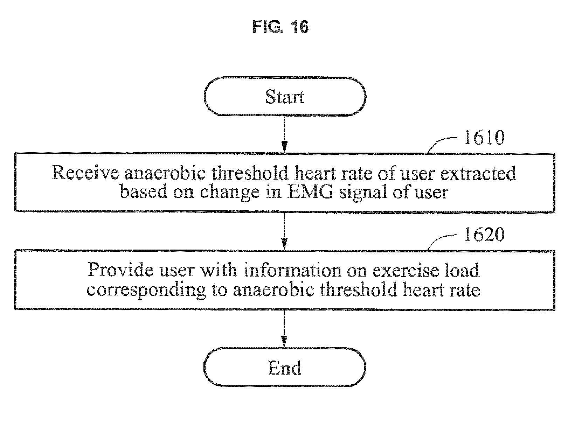

FIG. 16 illustrates an example of an exercise information providing method. The operations in FIG. 16 may be performed in the sequence and manner as shown, although the order of some operations may be changed or some of the operations omitted without departing from the scope of the illustrative examples described. Many of the operations shown in FIG. 16 may be performed in parallel or concurrently. The above descriptions of FIGS. 1-15, is also applicable to FIG. 16. Thus, the above description may not be repeated here.

Referring to FIG. 16, in operation 1610, an exercise information providing apparatus acquires an anaerobic threshold heart rate of a user based on a change in an EMG signal of the user. In operation 1620, the exercise information providing apparatus provides the user with information on an exercise load corresponding to the anaerobic threshold heart rate.

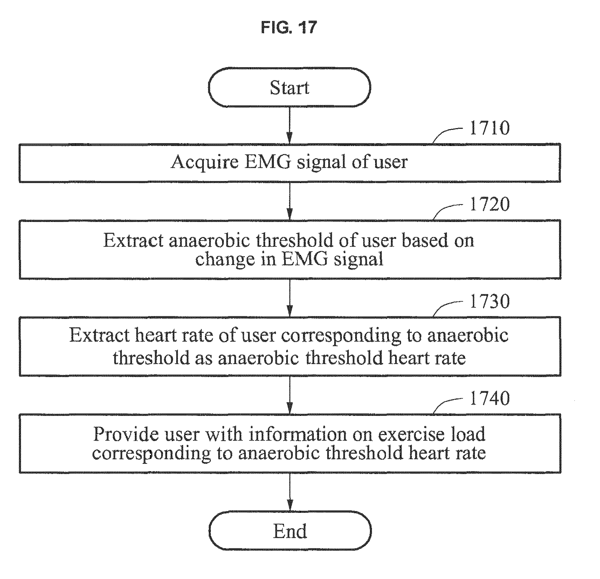

FIG. 17 illustrates another example of an exercise information providing method. The operations in FIG. 17 may be performed in the sequence and manner as shown, although the order of some operations may be changed or some of the operations omitted without departing from the scope of the illustrative examples described. Many of the operations shown in FIG. 17 may be performed in parallel or concurrently. The above descriptions of FIGS. 1-16, is also applicable to FIG. 17. Thus, the above description may not be repeated here.

Referring to FIG. 17, in operation 1710, an exercise information providing apparatus acquires an EMG signal of a user. In operation 1720, the exercise information providing apparatus extracts an anaerobic threshold of the user, based on a change in the EMG signal. In operation 1730, the exercise information providing apparatus extracts a heart rate of the user corresponding to the anaerobic threshold as an anaerobic threshold heart rate. In operation 1740, the exercise information providing apparatus provides the user with information on an exercise load corresponding to the anaerobic threshold heart rate.

The apparatuses and units described herein may be implemented using hardware components. The hardware components may include, for example, controllers, sensors, processors, generators, drivers, and other equivalent electronic components. The hardware components may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The hardware components may run an operating system (OS) and one or more software applications that run on the OS. The hardware components also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a hardware component may include multiple processors or a processor and a controller. In addition, different processing configurations are possible, such a parallel processors or multi-core processors.

A hardware component may be, for example, a physical device that physically performs one or more operations, but is not limited thereto. Examples of hardware components include resistors, capacitors, inductors, power supplies, frequency generators, operational amplifiers, power amplifiers, low-pass filters, high-pass filters, band-pass filters, analog-to-digital converters, digital-to-analog converters, and processing devices. A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

The processes, functions, and methods described above can be written as a computer program, a piece of code, an instruction, or some combination thereof, for independently or collectively instructing or configuring the processing device to operate as desired. Software and data may be embodied permanently or temporarily in any type of machine, component, physical or virtual equipment, computer storage medium or device that is capable of providing instructions or data to or being interpreted by the processing device. The software also may be distributed over network coupled computer systems so that the software is stored and executed in a distributed fashion. In particular, the software and data may be stored by one or more non-transitory computer readable recording mediums. The non-transitory computer readable recording medium may include any data storage device that can store data that can be thereafter read by a computer system or processing device. Examples of the non-transitory computer readable recording medium include read-only memory (ROM), random-access memory (RAM), Compact Disc Read-only Memory (CD-ROMs), magnetic tapes, USBs, floppy disks, hard disks, optical recording media (e.g., CD-ROMs, or DVDs), and PC interfaces (e.g., PCI, PCI-express, Wi-Fi, etc.). In addition, functional programs, codes, and code segments for accomplishing the example disclosed herein can be construed by programmers skilled in the art based on the flow diagrams and block diagrams of the figures and their corresponding descriptions as provided herein.

While this disclosure includes specific examples, it will be apparent to one of ordinary skill in the art that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims.

**Claims**

1. An anaerobic threshold extraction apparatus, comprising:

   an electromyogram, EMG, signal acquirer configured to acquire an EMG signal; and
   an anaerobic threshold extractor configured to extract an anaerobic threshold based on a change in the EMG signal, the anaerobic threshold indicating a time when muscle fatigue occurs.

2. The apparatus of claim 1, wherein the EMG signal acquirer is configured to acquire the EMG signal from at least one EMG sensor.

3. The apparatus of claim 1 or 2, wherein the anaerobic threshold extractor is configured to extract the anaerobic threshold based on change in the EMG signal.

4. The apparatus of claim 3, wherein the EMG signal changes in response to an exercise load of a user gradually increasing to a threshold exercise load,

wherein the anaerobic threshold extractor is further configured to extract the anaerobic threshold, based on a change in the EMG signal during a period of time or a change in the EMG signal until the exercise load reaches the threshold exercise load.

5. The apparatus of claim 3, wherein the anaerobic threshold extractor is further configured to perform one of transmitting a control signal to an exercise equipment used by the user to gradually increase the exercise load to the threshold exercise load; informing a user to gradually increase an exercise load to the threshold exercise load using any one or any combination of a visual interface, an acoustic interface, and a haptic interface; extracting a frequency of the EMG signal at predetermined time intervals, and extracting the anaerobic threshold based on a change in the frequency.

6. The apparatus of claim 5, wherein the anaerobic threshold extractor is further configured to identify heart rates of the user from a heartbeat sensor, and to extract a heart rate corresponding to the anaerobic threshold as an anaerobic threshold heart rate, and/or
wherein the anaerobic threshold extractor is further configured to transmit at least one of the extracted anaerobic threshold and the anaerobic threshold heart rate to an external apparatus via a communication interface.

7. The apparatus of claim 5, wherein the extracted frequency comprises a median frequency or a mean frequency of the EMG signal.

8. The apparatus of claim 5, wherein the anaerobic threshold extractor is further configured to perform one of identifying when the muscle fatigue occurs, in response to the frequency being reduced by at least a set amount, and transforming the EMG signal using either a fast Fourier transform, FFT, or a short-time Fourier transform, STFT, and is further configured to extract the frequency from the transformed EMG signal.

9. The apparatus of claim 5, wherein the anaerobic threshold extractor is further configured to extract the anaerobic threshold based on an EMG signal corresponding to a largest reduction in a frequency of a plurality of EMG signals acquired from a plurality of EMG sensors.

10. The apparatus of claim 1 adapted for extracting an anaerobic threshold heart rate, further comprising:

a heartbeat sensor configured to sense a heart rate and to acquire a heartbeat signal; and
an anaerobic threshold heart rate extractor configured to extract a heart rate corresponding to the anaerobic threshold as an anaerobic threshold heart rate,
wherein the EMG signal acquirer is an EMG sensor configured to sense an action potential of a muscle and to acquire the EMG signal.

11. A method of providing exercise information, comprising the step of:

acquiring, by an anaerobic threshold heart rate acquirer, an anaerobic threshold heart rate based on a change in an electromyogram, EMG, signal; and
providing, by an information providing unit, information on an exercise load corresponding to the anaerobic threshold heart rate.

12. The method of claim 11, further comprising:

receiving, by a heart rate identifier, a heartbeat signal from a heartbeat sensor, and identifying heart rates based on the heartbeat signal; and/ or
wherein the step of acquiring further comprises receiving an anaerobic threshold from an apparatus via a communication interface, and extracting a heart rate corresponding to the anaerobic threshold as the anaerobic threshold heart rate; and/or
wherein the step of providing information comprises evaluating an exercise efficiency based on a ratio of a heart rate to the anaerobic threshold heart rate, and providing the exercise efficiency.

13. The method of claim 11 or 12, wherein the step of providing information comprises informing a user on the exercise load using any one or any combination of a visual interface, an acoustic interface, and a haptic interface; and/or
wherein the step of providing information further comprises providing information on an exercise load to bring a heart rate within a threshold range of the anaerobic threshold heart rate, in response to the heart rate being outside the threshold range.

14. A computer-readable medium having instructions that, when performed by a processor, cause the processor to perform the steps of:

acquiring an electromyogram, EMG, signal from at least one EMG sensor;
determining the median or mean frequency of the acquired EMG signal; and
extracting an anaerobic threshold based on the frequency corresponding to a change in the EMG signal.

15. The computer-readable medium of claim 14, further comprising:

identifying heart rates from a heartbeat sensor; and
extracting a heart rate corresponding to the anaerobic threshold as an anaerobic threshold heart rate, and/ or wherein the determining of the median or mean frequency comprises transforming the EMG signal using a fast Fourier transform, FFT, or a short-time Fourier transform, STFT, and extracting the frequency from the transformed EMG signal.

FIG. 1

FIG. 2

## FIG. 3

```
                        ┌──────────┐
                        │  Start   │
                        └────┬─────┘
                             │                         ┌─ 310
              ┌──────────────▼──────────────────────┐
              │        Acquire EMG signal           │◄─┐
              └──────────────┬──────────────────────┘  │
                             │                ┌─ 320    │
              ┌──────────────▼──────────────────────┐  │
              │ Calculate median or mean frequency  │  │
              │          of the EMG signal          │  │
              └──────────────┬──────────────────────┘  │
                             │                ┌─ 330    │
              ┌──────────────▼──────────────────────┐  │
              │ Store the calculated median         │  │
              │ frequency or mean frequency         │  │
              └──────────────┬──────────────────────┘  │
                             │                          │
                         ╱───▼────╲         ┌─ 340      │
                      ╱             ╲                    │
                   ╱   EMG signal     ╲                 │
                ╱  is acquired prior to  ╲        Yes    │
              ╱  a point in time, or acquired ╲──────────┘
               ╲ at an exercise intensity equal╱
                ╲ to or less than an exercise  ╱
                 ╲        load               ╱
                      ╲               ╱
                         ╲───┬───╱
                             │ No          ┌─ 350
              ┌──────────────▼──────────────────────┐
              │ Calculate anaerobic threshold based │
              │ on the stored median frequency or   │
              │ mean frequency                      │
              └──────────────┬──────────────────────┘
                             │
                        ┌────▼─────┐
                        │   End    │
                        └──────────┘
```

FIG. 4

410

**FIG. 5**

**FIG. 6**

Raw EMG

FFT

FIG. 7

FIG. 8

FIG. 9

EP 3 009 068 A1

FIG. 10

FIG. 11

1111

Current speed is low.
Please run faster!

Current speed: 6.5km/h
Target speed: 7.5km/h

1110

1131

Exercise
mode
(for beginner)

1130

1132

1120

1121

♡160

1122

EP 3 009 068 A1

FIG. 12

Exercise history of Cheolsoo Kim

| Days | Date | Anaerobic exercise threshold | Average heart rate | Exercise efficiency |
|---|---|---|---|---|
| Last three days | September 5 | 126 | 125 | 99% |
| | September 3 | 126 | 128 | 101% |
| | September 1 | 125 | 121 | 97% |
| Month | September | 126 | 125 | 99% |
| | August | 124 | 123 | 99% |
| | July | 120 | 121 | 101% |

**FIG. 13**

FIG. 14

**FIG. 15**

```
          ┌──────────────────┐
          │      Start        │
          └──────────────────┘
                   │
                   ▼
    ┌──────────────────────────────────────┐  ╭─ 1510
    │      Acquire EMG signal of user        │
    └──────────────────────────────────────┘
                   │
                   ▼
    ┌──────────────────────────────────────┐  ╭─ 1520
    │   Extract anaerobic threshold of user, │
    │    based on change in EMG signal       │
    └──────────────────────────────────────┘
                   │
                   ▼
          ┌──────────────────┐
          │       End         │
          └──────────────────┘
```

FIG. 16

```
        ┌─────────────┐
        │    Start     │
        └──────┬──────┘
               │
               ▼                           ╭── 1610
 ┌──────────────────────────────────────────────┐
 │  Receive anaerobic threshold heart rate of    │
 │  user extracted based on change in EMG        │
 │  signal of user                               │
 └──────────────────┬───────────────────────────┘
                    │
                    ▼                      ╭── 1620
 ┌──────────────────────────────────────────────┐
 │  Provide user with information on exercise    │
 │  load corresponding to anaerobic threshold    │
 │  heart rate                                    │
 └──────────────────┬───────────────────────────┘
                    │
                    ▼
            ┌─────────────┐
            │     End      │
            └─────────────┘
```

**FIG. 17**

```
                    ┌─────────────┐
                    │    Start    │
                    └─────────────┘
                           │
                           ▼            ╱─ 1710
       ┌───────────────────────────────────────────┐
       │          Acquire EMG signal of user        │
       └───────────────────────────────────────────┘
                           │
                           ▼            ╱─ 1720
       ┌───────────────────────────────────────────┐
       │     Extract anaerobic threshold of user    │
       │       based on change in EMG signal        │
       └───────────────────────────────────────────┘
                           │
                           ▼            ╱─ 1730
       ┌───────────────────────────────────────────┐
       │  Extract heart rate of user corresponding  │
       │  to anaerobic threshold as anaerobic       │
       │          threshold heart rate              │
       └───────────────────────────────────────────┘
                           │
                           ▼            ╱─ 1740
       ┌───────────────────────────────────────────┐
       │  Provide user with information on exercise │
       │  load corresponding to anaerobic           │
       │          threshold heart rate              │
       └───────────────────────────────────────────┘
                           │
                           ▼
                    ┌─────────────┐
                    │     End     │
                    └─────────────┘
```

Europäisches
Patentamt

European
Patent Office

Office européen
des brevets

## EUROPEAN SEARCH REPORT

Application Number

EP 15 18 9870

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2006/079800 A1 (MARTIKKA MIKKO [FI] ET AL) 13 April 2006 (2006-04-13)<br>* paragraphs [0025], [0037] *<br>* paragraph [0039] - paragraph [0058] *<br>* paragraphs [0061], [0073] *<br>* paragraph [0077] - paragraph [0081] *<br>* figures *<br>----- | 1-7,10, 14<br><br>8,9,15 | INV.<br>A61B5/00<br>A61B5/0488<br>A61B5/22<br>A63B24/00<br><br>ADD.<br>A61B5/024 |
| X | QIN GUANG-XIA ET AL: "A non-invasive device for evaluating the anaerobic threshold of athletes (using EMG)", ENGINEERING IN MEDICINE AND BIOLOGY SOCIETY, 1988. PROCEEDINGS OF THE ANNUAL INTERNATIONAL CONFERENCE OF THE IEEE, IEEE, NEW YORK, NY, USA,<br>4 November 1988 (1988-11-04), page 1712, XP031931399,<br>DOI: 10.1109/IEMBS.1988.94936<br>ISBN: 978-0-7803-0785-8<br>* the whole document *<br>----- | 1-3 | |
| X<br><br>A | EP 2 732 758 A1 (SAMSUNG ELECTRONICS CO LTD [KR]) 21 May 2014 (2014-05-21)<br>* paragraph [0085] - paragraph [0104] *<br>* paragraph [0111] - paragraph [0125] *<br>* paragraph [0127] - paragraph [0132] *<br>----- | 1-3, 11-13<br>5-10 | TECHNICAL FIELDS SEARCHED (IPC)<br>A61B<br>A63B |

The present search report has been drawn up for all claims

| Place of search | Date of completion of the search | Examiner |
|---|---|---|
| The Hague | 7 March 2016 | Görlach, Tobias |

-/--

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ...................................................................................................
& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 15 18 9870

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SUN I KIM ET AL: "Mean Frequency Estimation Of Myoelectric Signal Using 2nd Order Maximum Entropy Method", ENGINEERING IN MEDICINE AND BIOLOGY SOCIETY, 1990., PROCEEDINGS OF THE TWELFTH ANNUAL INTERNATIONAL CONFERENCE OF THE IEEE PHILADELPHIA, PA, USA 1-4 NOV. 1990, NEW YORK, NY, USA,IEEE, US, 1 November 1990 (1990-11-01), pages 2208-2209, XP010035269, DOI: 10.1109/IEMBS.1990.692245 ISBN: 978-0-87942-559-3 | 8,9,15 | |
| A | * page 2208, right-hand column, paragraph 3 - page 2209, right-hand column, paragraph 1 * | 5-7,14 | |
| A | ISAO SASAKI ET AL: "Intelligent workload control of bicycle ergometer for the elderly based on individual physical work capacity", ELECTRONICS & COMMUNICATIONS IN JAPAN, PART II - ELECTRONICS., vol. 87, no. 9, 1 September 2004 (2004-09-01), pages 56-64, XP055254953, US ISSN: 8756-663X, DOI: 10.1002/ecjb.20116 * page 57, left-hand column, paragraph 4 - page 62, left-hand column, paragraph 1 * | 1-15 | |

-/--

TECHNICAL FIELDS
SEARCHED (IPC)

The present search report has been drawn up for all claims

| Place of search | Date of completion of the search | Examiner |
|---|---|---|
| The Hague | 7 March 2016 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 18 9870

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AKIRA NAGATA ET AL: "Anaerobic threshold determination by blood lactate and myoelectric signals.", JAPANESE JOURNAL OF PHYSIOLOGY., vol. 31, no. 4, 1 January 1981 (1981-01-01), pages 585-597, XP002755079, XX ISSN: 0021-521X, DOI: 10.2170/jjphysiol.31.585 * page 587, paragraph 4 - page 592, paragraph 1 * | 1-15 | |

-----

TECHNICAL FIELDS
SEARCHED    (IPC)

The present search report has been drawn up for all claims

| Place of search | Date of completion of the search | Examiner |
|---|---|---|
| The Hague | 7 March 2016 | Görlach, Tobias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

      ......................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 15 18 9870

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2016

| Patent document cited in search report | | Publication date | Patent family member(s) | | Publication date |
|---|---|---|---|---|---|
| US 2006079800 | A1 | 13-04-2006 | FI | 20050696 A | 02-01-2006 |
| | | | GB | 2415788 A | 04-01-2006 |
| | | | HK | 1085895 A1 | 14-08-2009 |
| | | | US | 2006079800 A1 | 13-04-2006 |
| EP 2732758 | A1 | 21-05-2014 | CN | 103815911 A | 28-05-2014 |
| | | | EP | 2732758 A1 | 21-05-2014 |
| | | | KR | 20140063330 A | 27-05-2014 |
| | | | US | 2014141937 A1 | 22-05-2014 |

EPO FORM P0459

For more details about this annex : see Official Journal of the European Patent Office, No. 12/82